Europäisches Patentamt

**European Patent Office**

Office européen des brevets

(19)

(11) Veröffentlichungsnummer: **0 014 979**
**B1**

(12) # EUROPÄISCHE PATENTSCHRIFT

(45) Veröffentlichungstag der Patentschrift:
**25.11.81**

(51) Int. Cl.³: **C 11 D 17/00, C 11 D 3/48**

(21) Anmeldenummer: **80100804.6**

(22) Anmeldetag: **18.02.80**

(54) **Verfahren zur Herstellung von Abspülblöcken für die Toilettenhygiene.**

(30) Priorität: **23.02.79 DE 2907029**

(43) Veröffentlichungstag der Anmeldung:
**03.09.80 Patentblatt 80/18**

(45) Bekanntmachung des Hinweises auf die Patenterteilung:
**25.11.81 Patentblatt 81/47**

(84) Benannte Vertragsstaaten:
**BE CH FR GB IT LU NL SE**

(56) Entgegenhaltungen:
**DE-A-2 407 947**
**DE-A1-2 910 955**
**US-A-3 970 576**

(73) Patentinhaber: **Henkel Kommanditgesellschaft auf Aktien, -Patentabteilung-**
**Postfach 1100 Henkelstrasse 67,**
**D-4000 Düsseldorf 1 (DE)**

(72) Erfinder: **Holdt, Berndt-Dieter, Gutenbergstrasse 45, D-4000 Düsseldorf (DE)**
Erfinder: **Menke, Ronald, Rosstrasse 20, D-4000 Düsseldorf (DE)**
Erfinder: **Praus, Gerd, Höppnerstrasse 80, D-4150 Krefeld (DE)**

BUNDESDRUCKEREI BERLIN

## Verfahren zur Herstellung von Abspülblöcken für die Toilettenhygiene

Abspülblöcke für die Toilettenhygiene bestehen im allgemeinen aus Mischungen, die neben Tensiden Desinfektions- und Bleichmittel, Komplexbildner, Salze, Säuren, Füllstoffe, Farb- und Geruchsstoffe, Plastifizierungsmittel u. dgl. enthalten können. Sie werden im Gieß- oder Extrudierverfahren hergestellt, was jedoch einen relativ hohen technischen Aufwand und/oder Energieverbrauch erfordert.

Der Erfindung liegt die Aufgabe zugrunde, ein Verfahren zur Herstellung von Abspülblöcken für die Toilettenhygiene aus pulver- oder granulatförmigen Wirkstoffgemischen zu finden, das den beschriebenen Aufwand vermeidet. Eine weitere Aufgabe der Erfindung besteht darin, die Einarbeitung von temperatur- und preßempfindlichen Wirkstoffen bei der Herstellung derartiger Abspülblöcke zu ermöglichen.

Gegenstand der Erfindung ist gemäß ein Verfahren zur Herstellung von Abspülblöcken für die Toilettenhygiene aus Wirkstoffen bzw. Wirkstoffgemischen auf der Basis von Tensiden, Desinfektions- und Bleichmitteln, Salzen, Säuren, Komplexbildern, Füllstoffen, Farb- und Geruchsstoffen und gegebenenfalls weiteren, in derartigen Gemischen verwendeten Stoffen, dadurch gekennzeichnet, daß man eine in Pulver- oder Granulatform vorliegende Mischung folgender Zusammensetzung:

10 – 70 Gew.-% anionische und/oder nichtionische Tenside,
0 – 60 Gew.-% Desinfektions- oder Bleichmittel,
0 – 70 Gew.-% saure, alkalische oder neutrale Salze, feste anorganische oder organische Säuren und/oder Komplexbildner,
0 – 20 Gew.-% Füllstoffe,
0 – 10 Gew.-% Farbstoff- und Geruchsstoffe,
0 – 5 Gew.-% Bindemittel,
20 – 90 Gew.-% hydratwasserbindende anorganische Salze

mit Wasser in Berührung bringt und dadurch verfestigt.

Die Wirkstoffe oder Stoffgemische enthalten als wesentlichen Bestandteil feste bis wachsartige anionische und/oder nichtionische Tenside, wie Fettalkoholsulfate, Alkylsulfonate, Olefinsulfonate, Alkylbenzolsulfonate der Kettenlängen $C_{10} - C_{20}$ in Form ihrer Alkalisalze, Addukte von 20 – 100 Mol Ethylenoxid (EO) und/oder Propylenoxid (PO) an höhere Fettalkohole oder Alkylphenole der Kettenlängen $C_{10} - C_{20}$, Polyoxyethylen- oder Polyoxyethylen-Polyoxypropylenglykole der Molgewichte 4000 – 20 000 u. dgl. Für das Verfahren der Erfindung kommen nur solche Tenside in Frage, die wachsartigen bis festen Charakter besitzen und in Pulver- oder Granulatform überführt werden können. Besonders bevorzugt werden Dodecylbenzolsulfonat-Natriumsalz, Olefinsulfat-Natriumsalze der Kettenlängen $C_{14} - C_{18}$, Laurylsulfat-Natriumsalz sowie $C_{16} - C_{18}$ – Fettalkoholpolyglykolether – 30 – 70 EO, sowie Gemische dieser Tenside.

Zur Verbesserung der Struktur, zur Erhöhung der Reinigungswirkung, sowie zur Verbilligung können die Stoffgemische als weitere Bestandteile saure, alkalische oder neutrale Salze, feste anorganische oder organische Säuren und/oder Komplexbildner, wie z. B. Natriumsulfat, Natriumbisulfat, Natriumchlorid, Natriumcarbonat, Kaliumcarbonat, Amidosulfonsäure, Weinsäure, Malonsäure, Citronensäure, Bernsteinsäure, Natriumtripolyphosphat, Ethylen-diamino-tetraacetat-Natriumsalz und dergleichen enthalten. Desgleichen können Füllstoffe, wie hochvoluminöse Kieselsäure, wasserunlösliche Alkalialuminiumsilikate, Farb- und Geruchsstoffe, z. B. wasserlösliche Triphenylmethan- oder Triacrylmethan-Farbstoffe, ätherische Öle, sowie die als druckempfindlich bekannten mikroeingekapselten Parfümöle und so weiter, sowie Bindemittel, wie Polyvinylalkohol, Polyvinylpyrrolidon, Carboxymethylcellulose zur Verhütung des Verwitterns der Blöcke bei der Lagerung, zugesetzt werden. Die Bindemittel können auch dem zur Verfestigung der Abspülblöcke benötigten Wasser zugegeben werden.

Sofern die Abspülblöcke Desinfektions- und Bleichwirkung besitzen sollen, werden ihnen entsprechende Wirkstoffe, wie z. B. quaternäre Ammoniumverbindungen, Chlorphenole und so weiter, zugesetzt. Insbesondere kommen hierfür aber Chlor- oder Sauerstoff abspaltende Mittel, wie Chlorcyanurat, Calciumhypochlorid, Natriumpercarbonat oder Natriumperborat in Betracht. Das Verfahren der Erfindung ermöglicht den Einsatz derartiger, preß- oder temperaturempfindlicher Wirkstoffe.

Ein wesentlicher Bestandteil der Stoffgemische sind hydratwasserbindende anorganische Salze oder Salzgemische, die in vollständig oder zum Teil entwässerter Form vorliegen. Als solche kommen in Frage Soda, Natriumsulfat, Natriumhydrogensulfat, Natriumpyrophosphat, Trinatriumphosphat, Natriumborat, Magnesiumsulfat, Zinkchlorid und dergleichen.

Vorzugsweise werden Salzgemische verwendet, die aus Kostengründen einen Anteil an Natriumsulfat enthalten. Reines Natriumsulfat ist wegen des niedrigen Schmelzpunktes seines Hydrates und seiner Verwitterungsneigung weniger geeignet. Beispielsweise bestehen geeignete

Salzmischungen aus

30 – 60 Gew.-% Natriumsulfat,
10 – 40 Gew.-% Borax,
10 – 30 Gew.-% Natriumhydrogensulfat

in wasserfreier Form.

Geeignete Gemische zur Herstellung von Abspülblöcken enthalten 20 – 90, vorzugsweise 30 – 60 Gew.-% an hydratwasserbindenden Salzen. Die Zusammensetzung entspricht der folgenden Formulierung:

10 – 70 Gew.-% anionische und/oder nichtionische Tenside,
0 – 60 Gew.-% Desinfektions- oder Bleichmittel,
0 – 70 Gew.-% saure, alkalische oder neutrale Salze, feste anorganische oder organische Säuren und/oder Komplexbildner,
0 – 20 Gew.-% Füllstoffe,
0 – 10 Gew.-% Farb- und Geruchsstoffe,
0 – 5 Gew.-% Bindemittel,
20 – 90 Gew.-% hydratwasserbindende Salze.

Besteht das Gemisch ausschließlich aus hydratwasserbindenden Salzen und Tensiden, muß mindestens ein Anteil von 30 Gew.-% an hydratwasserbindenden Salzen verwendet werden.

Eine bevorzugte Zusammensetzung für desinfektionsmittelfreie Abspülblöcke entspricht der folgenden Formulierung:

40 – 60 Gew.-% anionische und/oder nichtionische Tenside,
0 – 40 Gew.-% saure anorganische Salze,
0 – 40 Gew.-% Komplexbildner,
0 – 5 Gew.-% Farb- und Geruchsstoffe,
20 – 60 Gew.-% hydratwasserbindende Salze.

Für Desinfektionsmittel enthaltende Abspülblöcke wird folgende Rezeptur bevorzugt:

10 – 40 Gew.-% anionische und/oder nichtionische Tenside,
30 – 70 Gew.-% Desinfektions- oder Bleichmittel,
0 – 40 Gew.-% Komplexbildner,
0 – 5 Gew.-% Farb- und Geruchsstoffe,
20 – 60 Gew.-% hydratwasserbindende Salze.

Die Herstellung fester Abspülblöcke erfolgt dadurch, daß man die vorgenannten, in Pulver- oder Granulatform vorliegenden Stoffmischungen mit Wasser in Berührung bringt. Das Verfahren kann in verschiedener Weise durchgeführt werden. So kann man die Mischungen in geeignete Formen oder Behälter füllen und eine vorausbestimmte Wassermenge zugeben. Diese beträgt zweckmäßig 20 – 70% der zur vollständigen Hydratbildung erforderlichen theoretischen Wassermenge. Größere Wassermengen führen zu vergrößerter Aushärtung bzw. zu oberflächlichem Anlösen der Formkörper.

Die Aushärtungszeit richtet sich nach der Zusammensetzung der Formkörper und nach der zugefügten Wassermenge. Sie beträgt in günstigen Fällen wenige Minuten. Nach dem Aushärten können die Formkörper ohne besondere Maßnahmen, wie Kühlen oder dergleichen, aus der Form herausgenommen und verpackt werden. Auf diese Weise lassen sich Würfel, Zylinder, Kugeln oder dergleichen unmittelbar herstellen, die sich als Einlegestücke für Toilettenbecken oder als Nachfüllstücke für Einhängevorrichtungen für Wasserkästen oder dergleichen eignen. Das Gewicht der Körper beträgt zweckmäßig etwa 20 bis 200 g.

Ein Entnehmen der Formstücke aus der Form erübrigt sich, wenn das Formteil gleichzeitig als Verpackung dienen soll. Man füllt in diesem Falle in dünnwandige Kunststoffbehälter, z. B. Hülsen Becher oder Kegel aus Polystyrol, Polyethylen, Polypropylen oder PVC ab, die nach der Wasserzugabe gegebenenfalls mit einer Schutzkappe oder einem durchlöcherten Deckel verschlossen werden. Vorzugsweise kommen hierzu Dosierbehälter gemäß Gebrauchsmuster G 7 528 861 oder DE-OS 2 849 660 in Frage, die nur zu etwa 80% ihrer Höhe befüllt werden und mit Öffnungen für den Wasserzutritt und einem seitlichen Auslaufloch versehen sind. Die so erhaltenen Körper werden zusammen mit dem Formteil, das auch mit einer Einhängevorrichtung versehen sein kann, in WC-Spülkästen eingesetzt.

Ein weiterer Weg zur Herstellung der Abspülblöcke besteht darin, daß man die pulver- oder granulatförmigen Mischungen in poröse Behälter oder Beutel aus Kunststoff, Textilgewebe, wasserfest imprägniertem Papier oder dergleichen einfüllt, diese verschließt, und den Härtungsvorgang erst durch Einhängen oder Einlegen dieser Behälter oder Beutel in den Spülkasten oder in das Toilettenbecken vornimmt. Vorzugsweise kann man statt eines porösen Beutels eine verschweißbare wasserlösliche Folie verwenden, die sich bei Kontakt mit der Spülflüssigkeit auflöst unter gleichzeitiger Aushärtung des Inhalts. Schließlich können die Mischungen auch zum Nachfüllen von Dosierbehältern oder dergleichen, die mit dem Spülwasser in Berührung kommen, verwendet werden, wobei Aushärtung erfolgt.

Das beanspruchte Verfahren vermeidet apparative Aufwendungen, zum Beispiel für Extruder oder Pressen, sowie die beim Gießverfahren zum Schmelzen und Kühlen benötigte Energie. Es ermöglicht ferner den problemlosen Einsatz temperatur- und preßempfindlicher Wirkstoffe, beispielsweise von chlorabspaltenden Desinfektionsmitteln. Von Vorteil ist weiterhin das hohe spezifische Gewicht der mit der Erfindung hergestellten Körper, wodurch eine feste und stabile Lage, zum Beispiel am Grunde des Spülkastens erreicht wird. Insbesondere gegen Ende der Benutzungsdauer kann sonst ein unerwünschtes Aufschwimmen der spezifisch leichten Kunststoffbehältnisse erfolgen. Demgegenüber bereitet es Schwierigkeiten, nach dem Gießverfahren Körper mit hohem spezifischem Gewicht herzustellen, da größere Mengen an anorganischen Salzen, Füllstoffen und dergleichen zum Absitzen in der Schmelze neigen.

Von praktischer Bedeutung ist auch die Möglichkeit, die Abspülgeschwindigkeit der Blöcke durch das Mengenverhältnis von hydratwasserbindenden Salzen zu den übrigen Bestandteilen zu regulieren. Ein höherer Anteil an hydratwasserbindenden Salzen führt zu einer Herabsetzung der Abspülgeschwindigkeit und damit zu einer höheren Lebensdauer der Abspülblöcke. Üblicherweise sollen die Blöcke 300 bis 600 Spülungen aushalten.


Beispiele


1. Herstellung eines Abspülblockes mit Dosierbehälter und Aufhängevorrichtung


Ein pulverförmiges Gemisch aus

| Dodecylbenzolsulfonat-Na | | 35,0 Gew.-% |
|---|---|---|
| blauer Triphenylmethanfarbstoff | | 2,5 Gew.-% |
| Natriumsulfat | | 37,5 Gew.-% |
| Borax | wasserfrei | 12,5 Gew.-% |
| Natriumbisulfat | | 12,5 Gew.-% |

wird in ein ca. 120 ml fassendes zylindrisches Behältnis aus Polypropylen (Höhe 90 mm, Durchmesser 50 mm) entsprechend Gebrauchsmuster G 7 528 861 abgefüllt und über eine Dosiervorrichtung 25 ml Wasser zugegeben. Nach erfolgter Blockbildung (ca. 20 Minuten) wird ein durchlöcherter Deckel mit Aufhängevorrichtung aufgesetzt. Das Behältnis ist zum Einhängen in WC-Wasserkästen geeignet. Die Wirkstoffabgabe erfolgt über eine seitlich angebrachte 2 mm große Öffnung.


2. Herstellung eines Abspülblockes mit Dosierbehälter ohne Aufhängevorrichtung


Ein pulverförmiges Gemisch aus

| Dodecylbenzolsulfonat-Na | | 12,5 Gew.-% |
|---|---|---|
| $C_{15} - C_{18}$-Olefinsulfonat-Na | | 6,25 Gew.-% |
| wasserlöslicher blauer Farbstoff | | 2,5 Gew.-% |
| Natriumsulfat | | 53,75 Gew.-% |
| Borax | wasserfrei | 12,5 Gew.-% |
| Natriumbisulfat | | 12,5 Gew.-% |

wird in ein hohlkegelförmiges, ca. 130 ml fassendes Behältnis aus PVC (Höhe 70 mm, Einfüllöffnung 30 mm) gemäß DE-OS 2 849 660.8 abgefüllt und über eine Dosiervorrichtung 25 ml Wasser zugegeben. Nach erfolgter Blockbildung (ca. 20 Minuten) kann sofort in Kartons verpackt werden.

Die Behältnisse werden in WC-Wasserkästen eingelegt, wo sie infolge ihres hohen spezifischen Gewichtes zu Boden sinken und dort eine stabile aufrechte Lage einnehmen. Die Dosierung des Wirkstoffs erfolgt bei jedem Spülvorgang über eine seitlich angebrachte 3 mm große Öffnung.

### 3. Herstellung eines Abspülblockes mit Behältnis für Toilettenbecken

Ein pulverförmiges Gemisch aus

| | |
|---|---|
| Dodecylbenzolsulfonat-Na | 23,5 Gew.-% |
| $C_{16}-C_{18}$-Fettalkoholpolyglykol-ether 50 EO | 6,0 Gew.-% |
| Duftstoff (Citrusaroma) | 0,5 Gew.-% |
| Borax, wasserfrei | 70,0 Gew.-% |

wird in ein schalenförmiges, ca. 40 ml fassendes Kunststoffbehältnis abgefüllt und 8 ml Wasser zugegeben. Das Behältnis ist mit einem Wulstrand versehen, um das Herausfallen des Abspülblockes nach dem Erhärten (ca. 20 Minuten) zu verhindern. Mittels einer Aufhängevorrichtung kann das Behältnis in WC-Becken eingehängt werden.

### 4. Herstellung eines Abspülblockes in einem wasserlöslichen Folienbeutel

Ein pulverförmiges Gemisch aus

| | | |
|---|---|---|
| Dodecylbenzolsulfonat-Na | | 10,0 Gew.-% |
| Ethylendiamin-tetraacetat | | 30,0 Gew.-% |
| blauer Farbstoff | | 5,0 Gew.-% |
| Tetranatriumpyrophosphat | | 15,0 Gew.-% |
| Natriumsulfat | wasserfrei | 20,0 Gew.-% |
| Natriumhydrogensulfat | | 20,0 Gew.-% |

wird über eine Beutelfüllanlage in Folienbeutel aus PVA portioniert und eingeschweißt. Die befüllten Beutel können direkt in den Wasserkasten eines WC's gegeben oder aber in das in Beispiel 1 beschriebene Dosierbehältnis eingelegt (Abmessung der Beutel: Länge 90 mm, Breite 45 mm, Inhalt ca. 100 g), oder in ein allseitig offenes, mit Stegen versehenes Kunststoffbehältnis gelegt (Länge 60 mm, Breite 14 mm, Inhalt ca. 40 g) und in WC-Becken eingehängt werden.

Die Blockbildung erfolgt erst im Wasserkasten bzw. im WC-Becken während des Auflösuns der PVA-Folie.

### 5. Herstellung eines Abspülblockes mit Bleichwirkung

Ein in Granulatform vorliegendes Gemisch aus

| | | |
|---|---|---|
| Dodecylbenzolsulfonat-Na | | 10,0 Gew.-% |
| Natriumperborat-Monohydrat | | 20,0 Gew.-% |
| Borax | wasserfrei | 20,0 Gew.-% |
| Magnesiumsulfat | | 50,0 Gew.-% |

wird über einer Beutelfüllanlage in perforierte Folienbeutel aus PVC eingefüllt und eingeschweißt. Die Beutel können in beliebiger Größe direkt in den Wasserkasten eines WC's gegeben oder aber entsprechend Beispiel 4 zum Nachfüllen von Dosierbehältern oder Einhängekörbchen verwendet werden. Die Blockbildung erfolgt im Wasserkasten bzw. in WC-Becken. Die Wirkstoffabgabe erfolgt durch die Perforationen des Beutels (ca. 0,5 mm Durchmesser).

### 6. Herstellung eines Abspülblockes mit Desinfektionswirkung

An Stelle des in Beispiel 5 beschriebenen Gemisches kann ein in Granulatform vorliegendes Gemisch aus

| | | |
|---|---|---|
| Dodecylbenzolsulfonat-Na | | 15,0 Gew.-% |
| Chlorcyanurat | | 40,0 Gew.-% |
| Natriumsulfat | wasserfrei | 35,0 Gew.-% |
| Borax | | 10,0 Gew.-% |

in gleicher Weise in Beutelform zum Nachfüllen von Dosiervorrichtungen bzw. zum Einlegen in WC-Wasserkästen verwendet werden. Die Mischung besitzt eine ausgezeichnete Desinfektionswirkung und verhindert das Entstehen unangenehmer Gerüche.

### 7. Herstellung von Abspülblöcken mit Desinfektionswirkung für Stehtoiletten

Ein pulverförmiges Gemisch aus

| | | |
|---|---|---|
| Dodecylbenzolsulfonat-Na | | 12,5 Gew.-% |
| p-Chlor-m-kresol-Na | | 37,5 Gew.-% |
| Magnesiumsulfat }| wasserfrei | 37,5 Gew.-% |
| Borax } | | 12,5 Gew.-% |

wird über eine Dosiervorrichtung in vorgefertigte Mulden einer Tiefziehfolie aus PVC (Einzelstückgewicht ca. 30 g) gegeben. Nach Zugabe von 8 ml Wasser pro Mulde erfolgt Aushärtung innerhalb von ca. 20 Minuten. Danach können die Körper aus den Formen entnommen und weiter konfektioniert werden. Es ist aber auch möglich, die Form mittels Folie zu verschließen und in dieser Form in den Handel zu bringen. Die Körper können dann vom Verbraucher bei Bedarf einzeln entnommen werden.

### 8. Nachfüll-Pulvergemisch zur Herstellung von Abspülblöcken während des Gebrauchs

Ein pulverförmiges Gemisch aus

| | | |
|---|---|---|
| Dodecylbenzolsulfonat-Na | | 15,0 Gew.-% |
| Laurylsulfat-Na | | 5,0 Gew.-% |
| $C_{16} - C_{18}$-Fettalkoholpolyglykol-ether$-35$ EO | | 5,0 Gew.-% |
| blauer Triacrylmethanfarbstoff | | 2,0 Gew.-% |
| Natriumsulfat } | | 43,0 Gew.-% |
| Borax } | wasserfrei | 15,0 Gew.-% |
| Natriumhydrogensulfat } | | 15,0 Gew.-% |

dient zur Befüllung eines 250 – 500 ml fassenden, mit Dosiervorrichtung versehenen Vorratsbehälters. Das Pulvergemisch dient zum Nachfüllen von Dosierbehältnissen oder schalenförmigen Einhängekörbchen gemäß Beispielen 1 oder 3. Die Blockbildung erfolgt bei Spülwasserkontakt.

## Patentansprüche

1. Verfahren zur Herstellung von Abspülblöcken für die Toilettenhygiene aus Wirkstoffen bzw. Wirkstoffgemischen auf der Basis von Tensiden, Desinfektions- und Bleichmitteln, Salzen, Säuren, Komplexbildnern, Füllstoffen, Farb- und Geruchsstoffen und gegebenenfalls weiteren, in derartigen Gemischen verwendeten Stoffen, dadurch gekennzeichnet, daß man eine in Pulver- oder Granulatform vorliegende Mischung folgender Zusammensetzung:

10 – 70 Gew.-% anionische und/oder nichtionische Tenside,
0 – 60 Gew.-% Desinfektions- oder Bleichmittel,
0 – 70 Gew.-% saure, alkalische oder neutrale Salze, feste anorganische oder organische Säuren und/oder Komplexbildner,
0 – 20 Gew.-% Füllstoffe,
0 – 10 Gew.-% Farb- und Geruchsstoffe,
0 – 5 Gew.-% Bindemittel,
20 – 90 Gew.-% hydratwasserbindende anorganische Salze

mit Wasser in Berührung bringt und dadurch verfestigt.

2. Verfahren nach Anspruch 1, dadurch gekennzeichnet, daß man eine Mischung folgender Zusammensetzung

40 – 60 Gew.-% anionische und/oder nichtionische Tenside,
0 – 40 Gew.-% saure anorganische Salze,
0 – 40 Gew.-% Komplexbildner,
0 – 5 Gew.-% Farb- und Geruchsstoffe,
20 – 60 Gew.-% hydratwasserbindende anorganische Salze

mit Wasser in Berührung bringt und dadurch verfestigt.

3. Verfahren nach Anspruch 1, dadurch gekennzeichnet, daß man eine Mischung folgender Zusammensetzung

10 — 40 Gew.-%  anionische und/oder nichtionische Tenside,
30 — 70 Gew.-%  Desinfektions- oder Bleichmittel,
 0 — 40 Gew.-%  Komplexbildner,
 0 — 5 Gew.-%  Farb- und Geruchsstoffe,
20 — 60 Gew.-%  hydratwasserbindende anorganische Salze

mit Wasser in Berührung bringt und dadurch verfestigt.

4. Verfahren nach Anspruch 3 unter Verwendung von Mischungen, die ein Chlor abspaltendes Desinfektionsmittel enthalten.

5. Verfahren nach Anspruch 3 unter Verwendung von Mischungen, die ein Sauerstoff abspaltendes Bleichmittel enthalten.

6. Verfahren nach Anspruch 1 — 5 unter Verwendung von Mischungen, die wachsartige bis feste anionische Tenside aus der Gruppe der Alkylbenzolsulfonate, Olefinsulfonate und Fettalkoholsulfate der Kettenlängen $C_{10} - C_{20}$ und/oder nichtionische Tenside aus der Gruppe der Ethylenoxid und/oder Propylenoxidaddukte an höhere Fettalkohole oder Alkylphenole der Kettenlängen $C_{10} - C_{20}$ enthalten.

7. Verfahren nach Anspruch 1 — 6 unter Verwendung von Mischungen, die als hydratwasserbindende anorganische Salze Natriumsulfat, Natriumhydrogensulfat, Natriumpyrophosphat, Trinatriumphosphat, Natriumborat und/oder Magnesiumsulfat in wasserfreier Form enthalten.

8. Verfahren nach Anspruch 7 unter Verwendung von Mischungen, die als hydratwasserbindende anorganische Salze ein Gemisch aus Natriumsulfat und einem anderen hydratwasserbindenden Salz in wasserfreier Form enthalten.

9. Verfahren zur Herstellung von Abspülblöcken, dadurch gekennzeichnet, daß man eine Mischung gemäß Anspruch 1 — 8 in einer Form mit 20 — 70% der zur vollständigen Hydratbildung erforderlichen theoretischen Wassermenge versetzt und aushärten läßt.

10. Verfahren zur Herstellung von Abspülblöcken, dadurch gekennzeichnet, daß man eine Mischung gemäß Anspruch 1 — 8 in einem Beutel aus wasserlöslichem oder wasserdurchlässigem Material einschließt und die Aushärtung durch in Kontakt bringen mit der Spülflüssigkeit einer Toilette vornimmt.

## Claims

1. A process for the production of flushing blocks for lavatory hygiene from active ingredients or mixtures of active ingredients based on surfactants, disinfectans and bleaches, salts, acids, complexing agents, fillers, dyes and fragrances and, optionally, other substances used in mixtures of the type in question, characterised in that a powder-form or granular mixture having the following composition:

10 to 70%  by weight of anionic and/or nonionic surfactants,
 0 to 60%  by weight of disinfectants or bleaches,
 0 to 70%  by weight of acid, alkaline or neutral salts,

solid inorganic or organic acids and/or complexing agents,

 0 to 20%  by weight of fillers,
 0 to 10%  by weight of dyes and fragrances,
 0 to 5%  by weight of binders,
20 to 90%  by weight of inorganic salts capable of binding water of hydration,

is brought into contact with water and thereby solidified.

2. A process as claimed in Claim 1, characterised in that a mixture having the following composition:

40 to 60%  by weight of anionic and/or nonionic surfactants,
 0 to 40%  by weight of acid inorganic salts,
 0 to 40%  by weight of complexing agents,
 0 to 5%  by weight of dyes and fragrances,
20 to 60%  by weight of inorganic salts capable of binding water of hydration,

is brought into contact with water and thereby solidified.

3. A process as claimed in Claim 1, characterised in that a mixture having the following composition:

10 to 40% by weight of anionic and/or nonionic surfactants,
30 to 70% by weight of disinfectants or bleaches,
0 to 40% by weight of complexing agents,
0 to 5% by weight of dyes and fragrances,
20 to 60% by weight of inorganic salts capable of binding water of hydration,

is brought into contact with water and thereby solidified.

4. A process as claimed in Claim 3 using mixtures containing a chlorine-releasing disinfectant.

5. A process as claimed in Claim 3 using mixtures containing an oxygen-releasing bleach.

6. A process as claimed in Claims 1 to 5 using mixtures containing wax-like to solid anionic surfactants from the group comprising alkyl benzene sulfonates, olefin sulfonates and fatty alcohol sulfates containing from 10 to 20 C-atoms and/or nonionic surfactants from the group comprising ethylene oxide and/or propylene oxide adducts with higher fatty alcohols or alkyl phenols containing from 10 to 20 carbon atoms.

7. A process as claimed in Claims 1 to 6 using mixtures containing sodium sulfate, sodium hydrogen sulfate, sodium prophosphate, trisodium phosphate, sodium borate and/or magnesium sulfate in anhydrous form as the inorganic salts capable of binding water of hydration.

8. A process as claimed in Claim 7 using mixtures containning a mixture of sodium sulfate and another salt capable of binding water of hydration in anhydrous form as the inorganic salts capable of binding water of hydration.

9. A process for the production of flushing blocks, characterised in that 20 to 70% of the theoretical quantity of water required for complete hydrate formation is added to a mixture of the type claimed in Claims 1 to 8 in a mould, after which the mixture is left to harden.

10. A process for the production of flushing blocks, characterised in that a mixture of the type claimed in Claims 1 to 8 is introduced into a bag of watersoluble or water-permeable material and hardened by contact with flushing water of a lavatory.

**Revendications**

1. Procédé de fabrication de blocs de rinçage pour l'hygiène des toilettes à partir de matières actives ou de mélanges de matières actives à base de tensioactifs, d'agents de désinfection et de blanchiment, de sels, d'acides, de formateurs de complexes, de charges, de colorants et de parfums et éventuellement d'autres substances utilisées dans de tels mélanges, caractérisé en ce qu'on met un mélange se présentant à l'état de poudre ou de granulat, de composition suivante:

10 – 70% en poids de tensioactifs anioniquès et/ou non ioniques,
0 – 60% en poids d'agents de désinfection ou de blanchiment,
0 – 70% en poids de sels acides, alcalins ou neutres, d'acides minéraux ou organiques solides et/ou de formateurs de complexes,
0 – 20% en poids de charges,
0 – 10% en poids de colorants et de parfums,
0 – 5% en poids d'agents liants,
20 – 90% en poids de sels minéraux fixateurs d'eau d'hydratation

en contact avec de l'eau et en ce qu'on le consolide ainsi.

2. Procédé selon la revendication 1, caractérisé en ce qu'on met un mélange de composition suivante:

40 – 60% en poids de tensioactifs anioniques et/ou non ioniques,
0 – 40% en poids de sels minéraux acides,
0 – 40% en poids de formateurs de complexes,
0 – 5% en poids de colorants et de parfums,
20 – 60% en poids de sels minéraux fixant l'eau d'hydratation

en contact avec de l'eau et en ce qu'on le consolide ainsi.

**0 014 979**

3. Procédé selon la revendication 1, caractérisé en ce qu'on met un mélange de composition suivante:

10—40% en poids de tensioactifs anioniques et/ou non ioniques,
30—70% en poids de désinfectants ou d'agents de blanchiment,
0—40% en poids de formateurs de complexes,
0—5% en poids de colorants et de parfums,
20—60% en poids de sels minéraux fixant l'eau d'hydratation

en contact avec de l'eau et en ce qu'on le consolide ainsi.

4. Procédé selon la revendication 3 avec utilisation de mélanges qui contiennent un agent désinfectant dégageant du chlore.

5. Procédé selon la revendication 3 avec utilisation de mélanges qui contiennent un agent de blanchiment dégageant de l'oxygène.

6. Procédé selon les revendications 1 à 5 avec utilisation de mélanges qui contiennent des tensioactifs anioniques cireux à solides du groupe des alcoylbenzène-sulfonates, oléfine-sulfonates et sulfates d'alcools gras de longuers de chaîne en $C_{10}-C_{20}$ et/ou des tensioactifs non ioniques du groupe des produits d'addition d'oxyde d'ethylène et/ou d'oxyde de propylène sur des alcools gras supérieurs ou des alcoylphénols ayant des longueurs de chaînes en $C_{10}-C_{20}$.

7. Procédé selon les revendications 1 à 6 avec utilisation de mélanges qui contiennent comme sels minéraux fixant l'eau d'hydratation du sulfate de sodium, de l'hydrogénosulfate de sodium, du pyrophosphate de sodium, du phosphate trisodique, du borate de sodium et/ou du sulfate de magnésium sous la forme anhydre.

8. Procédé selon la revendication 7 avec utilisation de mélanges qui contiennent en tant que sels minéraux fixant l'eau d'hydratation un mélange de sulfate de sodium et d'un autre sel fixant l'eau d'hydratation sous la forme anhydre.

9. Procédé de fabrication de blocs de rinçage, caractérisé en ce qu'on ajoute à un mélange selon les revendications 1 à 8 dans un moule 20 à 70% de la quantité d'eau théoriquement nécessaire pour la formation complète de l'hydrate et en ce qu'on laisse durcir.

10. Procédé de fabrication de blocs de rinçage, caractérisé en ce qu'on insére un mélange selon les revendications 1 à 8 dans un sac constitué en un metériau soluble dans l'eau ou perméable à l'eau et l'on exécute le durcissement complet par mise en contact avec le liquide de rinçage d'une toilette.

9